# EUROPEAN PATENT APPLICATION

(11) **EP 4 725 539 A1**
(43) Date of publication of application: **15.04.2026**
(21) Application number: 25208229.2
(22) Date of filing: 13.10.2025
(51) Int. Cl.: A61M 25/10, A61L 15/00, A61L 29/14

(54) **MEDICAL BALLOON REINFORCED WITH A BRAIDED CONSTRUCT OF A LOW-MELTING TEMPERATURE BONDING YARN AND A HIGH-STRENGTH YARN**

(30) Priority: 11.10.2024 US 202463706053 P; 09.10.2025 US 202519354100
(71) Applicant: Aran Biomedical Teoranta, County Galway, H91 C2NF (IE)
(72) Inventor: ASPEL, Brendan, Co. Kildare, W91 RYV8 (IE); KING, Dean, Co. Cavan, H91 F218 (IE); JONES, Michael, Co. Galway, H91 A6N7 (IE); HAYES, Gary, Co. Galway (IE)
(74) Representative: Mathys & Squire

(57) **Abstract**

A medical balloon comprises a braided construct (14) of a low melting temperature bonding yarn (26) and a high-strength reinforcing yarn (28). The braided structure (14) is contacted to the outer surface of a polymeric balloon serving as a scaffold and then post-processed in a reflow heating step which elevates the assembly above the melting temperature of the bonding yarn (26). The reflow heating step causes the bonding yarn (26) to melt and flow over, around and under the reinforcing yarn (28) and over and around the balloon. When the melted bonding yarn (26) is cooled to an ambient temperature, it bonds to the reinforcing yarn (28) and to the outer surface of the balloon.

## Description

### CROSS-REFERENCE TO RELATED APPLICATION

The present application claims priority to U.S. provisional application Serial No. 63/706,053, filed on October 11, 2024, as well as US Patent Application Serial No. 19/354,100, filed on October 9, 2025.

### FIELD OF THE INVENTION

This invention relates to medical balloons for high pressure vascular angioplasty.

### BACKGROUND OF THE INVENTION

High pressure balloon catheters are used in vascular angioplasty to break open heavily calcified or highly stenotic lesions, including coronary, peripheral, neurovascular and structural heart applications. One example is the use of high-pressure balloons for vascular access to arteriovenous shunts used in kidney dialysis where the patient will need repeated access to the shunt. Arteriovenous shunts can become highly stenotic over time and require high pressure balloons to open. Other applications include peripheral angioplasty balloons to break open highly calcified vascular lesions, valvuloplasty to break open calcified heart valves in preparation for valve repair and replacement, and high pressure non-compliant balloons used in coronary angioplasty and for coronary stent placement.

A known medical balloon forming process involves blowing a thin film of polymer material (or multiple layers of different polymers) into the shape of a balloon in a thermal forming process. The state of the art for high pressure balloons involves braiding a textile material that is then contacted to the blown film balloon. However, these conventional balloons are still prone to bursting at high pressure, potentially resulting in the loss of balloon material within the venous system of the patient.

For example, PCT Pub. No. WO 2006/034396 discloses surgical balloons that are made from woven, braided, or knitted yarns and metal filaments under tension. Suitable yarns include polyethylene teraphathalate (PET)/polyester), for example, the yarn marketed under the trademark DACRON^{®} by E. I. Du Pont de Nemours and Company, Wilmington, DE, nylon, liquid crystal polymer fibers, for example the yarns marketed under the trademark VECTRAN^{®} by Celanese Acetate, LLC, Kronberg/Taunus, Germany, and marketed under the trademark KEVLAR^{®} by E. I. Du Pont de Nemours and Company, Wilmington, DE, SPECTRA^{®}, metal filaments such as steel, nickel titanium alloy filaments (e.g., Nitinol), nickel-cobalt alloy filaments marketed under the trademark MP35N^{®} by Magellan Industrial Trading Company, Inc., Westport, CT, and combinations thereof. The yarns and metal filaments can be impregnated, coated, dipped, or otherwise mixed with polymers to minimize fluid leakage through the balloon. However, the woven, braided, or knitted yarns and metal filaments, whether coated, dipped, or otherwise mixed with polymers, do not provide acceptable high-pressure burst tolerance.

U.S. Pub. No. 2003/0204235 to Edens et al. discloses several exemplary implantable tubular textile prostheses that are suitable for use as endovascular grafts, balloon catheters, meshes, vascular patches, hernia plugs, stent-graft composites, blood filters, self-expanding stents, and balloon expandable stents, and the like. The tubular textile prostheses comprise a biocompatible fabric made from cold drawn PTFE yarns having a substantially uniform denier and high molecular orientation. However, the cold drawn PTFE yarns do not consistently provide the tubular textile prosthesis with acceptable high-pressure burst tolerance.

U.S. Pub. No. 2005/0271844 to Mapes et al. discloses medical balloons that are used in percutaneous transluminal coronary angioplasty procedures, and as cutting balloons. The balloons are formed from at least one polymeric balloon layer having inner and outer surfaces. A silk fiber web is provided over at least a portion of the inner surface, the outer surface or both inner and outer surfaces of the polymeric balloon. However, the silk fiber reinforcement web for the polymeric balloon layer does not always provide acceptable high-pressure burst tolerance for the product medical balloon.

In that respect, there is an ongoing need for a new design for a medical balloon so that the balloon has improved high-pressure burst tolerance. That is so the novel medical balloon is acceptable for use in vascular angioplasty to break open heavily calcified or highly stenotic lesions, peripheral angioplasty to break open highly calcified vascular lesions, valvuloplasty to break open calcified heart valves in preparation for valve repair and replacement, and as a high pressure non-compliant balloon used in coronary angioplasty and for coronary stent placement.

### SUMMARY OF THE INVENTION

Aspects and embodiments of the present invention are set out in the appended claims.

The present invention describes a novel design approach for a medical balloon. The medical balloon is comprised of a braided construct that is contacted to the outer surface of a polymeric balloon serving as a scaffold for the construct. The braided construct integrates two typical polymeric yarns into a braided configuration such as a braided sleeve around a low-profile balloon. One of the polymeric yarns is termed a bonding yarn because it has a relatively low melting temperature profile. Polyester or PET (polyethylene terephthalate) including copolyester and polyamide including co-polyamide are exemplary bonding yarns. The other yarn is termed a reinforcing yarn because it has a relatively high-strength, low-elongation profile. Ultra-high molecular weight polyethylene (UHMWPE), liquid crystal polymer (LCP) and the para-aramid marketed as TECHNORA^{®} by Teijin Limited are exemplary reinforcing yarns.

This braided construct is subsequently contacted to the outer surface of a polymeric balloon serving as a scaffold for the construct and the assembly is then heated in a reflow process which elevates the assembly above the melting temperature of the bonding yarn. It is preferred that the bonding yarn used in the braided construct has a melting temperature that is lower than that of both the balloon and the reinforcing yarn. The reflow process causes the bonding yarn to melt and flow over, around and under the reinforcing yarn and over and around the balloon. When the melted bonding yarn is cooled to an ambient temperature, it bonds to the surface of the reinforcing yarns and to the outer surface of the balloon.

The resulting medical balloon has improved burst strength at high pressure, which makes the present invention balloon particularly advantageous in vascular angioplasty to break open heavily calcified or highly stenotic lesions, peripheral angioplasty to break open highly calcified vascular lesions, valvuloplasty to break open calcified heart valves in preparation for valve repair and replacement, and as a high pressure non-compliant balloon used in coronary angioplasty and for coronary stent placement.

These and other aspects of the present invention will become more apparent to those skilled in the art by reference to the following description and the appended drawings.

### BRIEF DESCRIPTION OF THE DRAWINGS

FIG. 1 is an elevational view of a medical balloon 10 according to the present invention.
FIG. 2 is an elevational view of a balloon 12 serving as a scaffold prior to the balloon being contacted with a braided construct 14 (FIG. 3) according to the present invention.
FIG. 3 is an elevational view of a braided construct 14 comprising a low melting temperature bonding yarn 26 that is braided with a high-strength, low-elongation reinforcing yarn 28 prior to the construct 12 being contacted to the outer surface of the balloon 12 shown in FIG. 2.

### DETAILED DESCRIPTION OF THE PREFERRED EMBODIMENTS

As used in this specification, the term "reflow" means that the polymeric material comprising the bonding yarn has been heated above its melting temperature so that the polymeric material softens, flows, and mechanically connects to adjacent bonding yarn, to adjacent polymeric reinforcing yarn and to the outer surface of a polymeric balloon serving as a scaffold without the polymeric materials comprising the reinforcing yarn and the balloon having been melted.

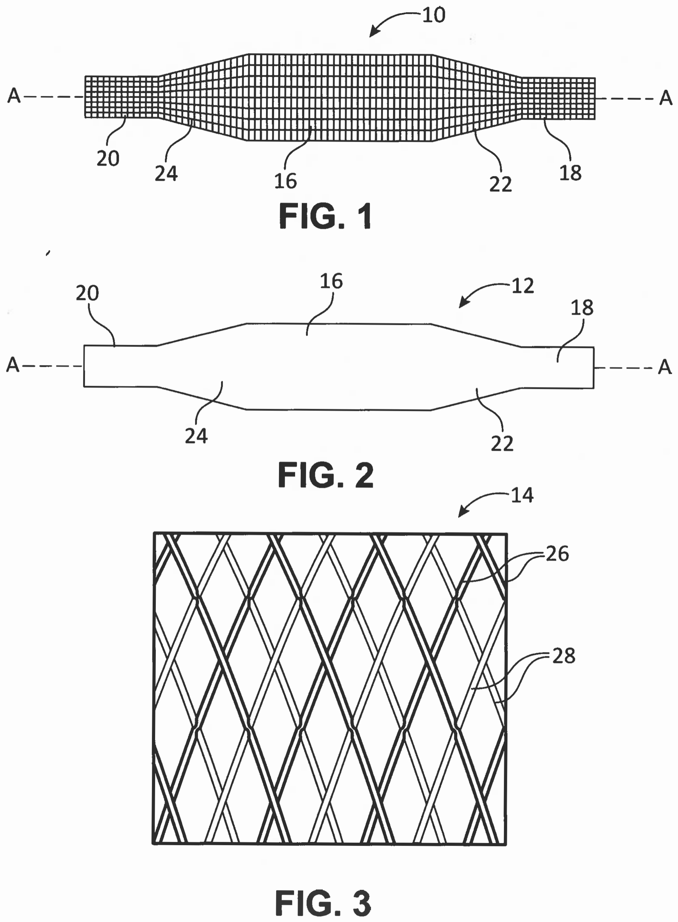

Turning now to the drawings, FIG. 1 shows a first embodiment of a medical balloon 10 according to the present invention. The medical balloon 10 is comprised of a balloon 12 of a base polymeric material (FIG. 2) serving as a scaffold for a braided construct 14 (FIG. 3). A typical medical balloon 10 comprising the braided construct 14 contacted to the outer surface of the balloon 12 may have a total wall thickness of about 20 to about 30 microns.

The balloon 12 is made from a blown polymeric material having a tubular body comprising an enlarged open central section 16 extending along a longitudinal axis A-A to opposed narrow open-ended sections 18, 20 that are adapted to fit over a catheter shaft (not shown). Tapered sections 22 and 24 extend axially from the central section 16 to the respective open-ended sections 18 and 20. The opposed open-ended sections 18, 20 have desired diameters to suit the application for which the medical balloon 10 is intended to be used.

The balloon 12 may be constructed in a variety of ways and comprise at least a first polymeric layer which defines the shape of the medical balloon 10. The first layer is formed from any suitable polymeric material known in the art including thermoplastic materials and thermosetting materials such as moisture curable and radiation curable monomers, oligomers, and polymers, as well as elastomers and non-elastomers.

Exemplary elastomeric materials that are suitable for the balloon 12 comprise, but are not limited to, elastomeric block copolymers including those having styrene end blocks and diene mid blocks such as styrene-ethylene-butylene-styrene (SEBS) block copolymers disclosed in U.S. Pat. No. 5,112,900 to Buddenhagen et al., which is incorporated herein by reference. Other suitable block copolymeric elastomers include, but are not limited to, styrene-isoprene-styrene (SIS) block copolymer, and styrene-butadiene-styrene (SBS) block copolymer.

Suitable polyamides for the balloon 12 include nylon, and copolymers thereof such as poly (ether-block-amides) available under the tradename of PEBAX^{®} from Atofina Chemicals, Philadelphia, Pa.

Examples of non-elastomeric materials that are suitable for the balloon 12 include, but are not limited to, polyolefins, polyesters, copolyesters, polyethers, polyamides, polyurethanes, polyimides, copolymers and terpolymers thereof. Examples of elastomeric copolyesters include, but are not limited to, poly(ester-block ether) elastomers, and poly(ester-block-ester) elastomers. Poly(ester-block-ether) elastomers are available under the tradename of HYTREL^{®} from DuPont de Nemours & Co. and consist of hard segments of polybutylene terephthalate and soft segments based on long chain polyether glycols. These polymers are also available from DSM Engineering Plastics under the tradename of ARNITEL^{®}.

Non-elastomeric polyesters and copolymers may also be used to construct the balloon 12. These materials include polyethylene terephthalates and polybutylene terephthalates.

Once a suitable polymeric material has been selected for the balloon 12 based on the specific application that the product medical balloon 10 is intended, the balloon 12 is formed using any conventional technique known in the art. An exemplary balloon forming technique is described in U.S. Pat. No. 4,490,421 to Levy, which is incorporated herein by reference. The disclosed method includes the basic steps of extruding a tubular balloon preform, placing the tubular preform in a balloon mold, and expanding the tubular preform into the desired balloon configuration in the mold. Other processing steps may include stretching and radial orientation of the balloon material as well as annealing and heat setting.

If desired, prior to contact with the braided construct 14, the thusly formed balloon 12 may be further provided with a polymeric coating having a thickness of about 5 microns to about 10 microns. For example, the outer surface of the balloon 12 may be coated with polyurethane by a dispersion process, such as by spraying the polyurethane onto the balloon 12. Once the polyurethane has dried or cured, the balloon 12 may be everted (turned inside out), and the inner surface of the balloon, now facing outwards, may be coated with polyurethane by repeating the dispersion process. Once the second coating layer has dried or cured, the resulting balloon 12 may be everted once again.

As shown in FIG. 3, the braided construct 14 comprises at least one relatively low melting temperature bonding yarn 26 that is braided with at least one relatively high-strength, low-elongation reinforcing yarn 28. In various embodiments, the braided construct 14 may comprise the bonding yarns 26 and the reinforcing yarns 28 individually as monofilament yarns, multifilament yarns (which may be heat set in a twisted condition or not), or a combination of both.

Suitable relatively low melting temperature bonding yarns 26 include:
the copolyester designated GRILON KE-60 having a melting temperature of about 55°C. to about 65°C.,
the copolyester designated KE-60 having a melting temperature of about 60°C.,
the copolyimide designated K-85 having a melting temperature of about 85°C.,
the copolyester designated GRILON K-85 having a melting temperature of about 82°C. to about 92°C.,
the copolyimide designated K-110 having a melting temperature of about 110°C.,
the copolyester designated KE-135 having a melting temperature of about 135°C.,
the copolyimide designated K-140 having a melting temperature of about 140°C.,
the copolyester designated KE-160 having a melting temperature of about 160°C.,
Nylon 12 having a melting temperature of about 178°C., and
PET (polyethylene terephthalate) having a melting temperature of about 260°C.

Suitable relatively high-strength, low-elongation reinforcing yarns 28 are selected from:
UHMWPE (ultra-high molecular weight polyethylene),
LCP (liquid crystal polymer), and
The aramid marketed by Teijin Limited under the brand name TECHNORA^{®}.

Fine gauges of implantable grades of 316L stainless-steel, titanium (Ti-6Al-4V) and Co-Cr are also suitable as relatively high-strength, low-elongation reinforcing yarns.

For example, mixing PET (melting temperature of about 260°C.) with Nylon 12 (melting temperature of about 178°C.) fibers as a composite yarn would enhance bonding to one of the enumerated reinforcing yarns 28 because Nylon 12 has a relatively low melting temperature compared with PET, which would provide additional strength to the product medical balloon 10.

In addition to the low melting temperature bonding yarn 26 and the high-strength, low-elongation reinforcing yarn 28 being individual yarns that are subsequently braided to form the construct, the bonding yarn 26 and the reinforcing yarn 28 may also be twisted or plied into a singular yarn structure prior to braiding.

Thus, the braided construct 14 comprising at least one low melting temperature bonding yarn 26 braided with at least one high-strength, low-elongation reinforcing yarn 28 is then contacted to the outer surface of the balloon 12. When contacted to the balloon 12, the braided construct 14 may have the braided bonding yarns 26 and the reinforcing yarns 28 running both axially around the circumference and longitudinally along the axis A-A extending along the length of the balloon 12.

Preferably the bonding yarn 26 and the reinforcing yarn 28 in the braided construct 14 are oriented in a range of from about 100° to about 140°, preferably at about 120°, with respect to each other. This relative orientation is preferred in contrast to yarns that are oriented perpendicularly. Importantly, a braided construct 14 where the bonding yarns 26 and the reinforcing yarns 28 are oriented at about 120° with respect to each other provided better reinforcement in the tapered sections 22 and 245 of the product medical balloon 10. The braided construct 14 where the bonding yarns 26 and the reinforcing yarns 28 are oriented at about 120° with respect to each other also provided improved reinforcement to the balloon 12 in the circumferential direction.

Some embodiments of the medical balloon 10 may vary the braid configuration or cover factor of the braided construct 14 across the length of the balloon 12. For example, the braided construct 14 in contact with the tapered sections 22, 24 of the balloon 12 may have increased thicknesses to provide enhanced reinforcement to those regions. In another embodiment, the ratio of reinforcing yarn 28 to bonding yarn 26 may be altered to vary the final assembly thickness, degree of attachment, or level of reinforcement. This may be particularly important in the tapered sections 22, 24 where stress and strain during inflation of the medical balloon 10 is important to control.

In one preferred embodiment, the braided construct 14 is provided as a braided sleeve composed of a relatively high number of yarns where every 3^{rd} yarn is a low melting temperature bonding yarn 26 with the balance being reinforcing yarns 28. In another embodiment, the braided construct 14 is provided as a braided sleeve composed of a relatively high number of yarns where every 4^{th} yarn is a low melting temperature bonding yarn 26 with the balance being reinforcing yarns 28. In still another embodiment, the braided construct 14 is provided as a braided sleeve composed of a relatively high number of yarns where every 5^{th} yarn is a low melting temperature bonding yarn 26 with the balance being reinforcing yarns 28. Thus, the ratio of bonding yarns 26 to reinforcing yarns 28 in the braided construct 14 ranges from about 1:2 to 1:4, preferably about 1:3.

The braided construct 14 as a tubular sleeve is then slid over the balloon 12 and the assembly is subjected to a reflow process above the melting point of the bonding yarn 26. This causes the bonding yarn 26 to flow in and around the reinforcing yarn 28 at the interface with the balloon 12. Upon cooling, the reflowed bonding yarn 26 bonds the reinforcing yarn 28 to the outer surface of the balloon 12.

In any event, the assembly comprising the braided construct 14 contacted to the outer surface of the balloon 12 is subsequently heat-processed. The application of heat to the balloon 12/braided construct 14 elevates the assembly above the melting temperature of the at least one low melting temperature bonding yarn 26. This causes the bonding yarn 26 to melt and flow over, under and alongside the at least one reinforcing yarn 28 so that when the bonding yarn 26 cools to an ambient temperature, the bonding yarn 26 is bonded to the reinforcing yarn 28 and to the outer surface of the balloon 12. The heating step may, for example, be undertaken by heating a mandrel on which the balloon 12 is mounted. The heating step may also be performed in a convention oven or reflow machine.

If desired, the thusly constructed medical balloon 10 is coated with a polymeric material using a solution dispersion or dip coating method. The resulting medical balloon 10 offers a resilient mechanical structure with excellent compliance when inflated to high pressures, i.e., those ranging from about 30 ATM to about 50 ATM.

The invention is not limited to the embodiments described herein but can be amended or modified without departing from the scope of the present invention. In that respect, the scope of protection afforded the disclosed medical balloon 10 is defined by the appended claims.

## Claims

1. A medical balloon, comprising:
a) a balloon comprising a base polymeric material of a first melting temperature, the balloon having an outer surface; and
b) a braided construct contacting the outer surface of the balloon, wherein the braided construct comprises at least one bonding yarn of a second polymeric material having a second melting temperature braided with at least one reinforcing yarn of a third material having a third melting temperature, and wherein the second melting temperature of the bonding yarn is lower than the third melting temperature of the reinforcing yarn,
c) wherein the braided construct contacting the outer surface of the balloon is characterized as the second polymeric material comprising the bonding yarn having been reflowed to mechanically connect to the base polymeric material comprising the balloon and to the third material comprising the reinforcing yarn without the balloon and the reinforcing yarn having been melted.

2. The medical balloon of claim 1, wherein the third material comprising the reinforcing yarn and the base polymeric material comprising the balloon are not melted.

3. The medical balloon of claim 1 or claim 2, wherein the second melting temperature of the second polymeric material of the at least one bonding yarn is lower than the first melting temperature of the base polymeric material comprising the balloon and the third melting temperature of the third material of the at least one reinforcing yarn.

4. The medical balloon of any preceding claim, wherein the braided construct is a braided sleeve contacted to the outer surface of the balloon.

5. The medical balloon of any preceding claim, wherein the at least one bonding yarn and the at least one reinforcing yarn in the braided construct are in a ratio ranging from 1:2 to 1:4.

6. The medical balloon of any preceding claim, wherein the at least one bonding yarn and the at least one reinforcing yarn are oriented with respect to each other in the braided construct in a range of from about 100° to about 140°.

7. The medical balloon of any preceding claim, wherein the balloon comprises an enlarged central balloon section the meets opposed tapered balloon sections extend axially to respective open-ended balloon sections, and wherein the braided construct in contact with the tapered balloon sections has an increased thickness in comparison to the braided construct in contact with the enlarged central balloon section and the open-ended balloon sections.

8. The medical balloon of any preceding claim, wherein:
the base polymeric material comprising the balloon is selected from a styrene-ethylene-butylene-styrene (SEBS) block copolymer, a styrene-isoprene-styrene (SIS) block copolymer, a styrene-butadiene-styrene (SBS) block copolymer, nylon, a poly (ether-block-amides, a poly(ester-block ether) elastomer, a poly(ester-block-ester) elastomer, a polyethylene terephthalate, and a polybutylene terephthalate; and/or
the second polymeric material comprising the at least one bonding yarn is selected from a copolyester designated GRILON KE-60 having a melting temperature of about 55°C. to about 65°C., a copolyester designated KE-60 having a melting temperature of about 60°C., a copolyimide designated K-85 having a melting temperature of about 85°C., a copolyester designated GRILON K-85 having a melting temperature of about 82°C. to about 92°C., a copolyimide designated K-110 having a melting temperature of about 110°C., a copolyester designated KE-135 having a melting temperature of about 135°C., a copolyimide designated K-140 having a melting temperature of about 140°C., a copolyester designated KE-160 having a melting temperature of about 160°C., Nylon 12 having a melting temperature of about 178°C., and PET (polyethylene terephthalate) having a melting temperature of about 260°C; and/or
the third material comprising the at least one reinforcing yarn is selected from UHMWPE (ultra-high molecular weight polyethylene), LCP (liquid crystal polymer), an aramid, a fine gauge 316L stainless-steel, a fine gauge titanium (Ti-6Al-4V), and a fine gauge Co-Cr.

9. The medical balloon of any preceding claim, wherein the second polymeric material comprising the bonding yarn is PET having a melting temperature of about 260°C. mixed with Nylon 12 having a melting temperature of about 178°C.

10. A medical balloon, comprising:
a) a balloon comprising a base polymeric material of a first melting temperature, the balloon having an outer surface; and
b) a braided construct contacting the outer surface of the balloon, wherein the braided construct comprises at least PET (polyethylene terephthalate) as a bonding yarn braided with at least UHMWPE (ultra-high molecular weight polyethylene) as a reinforcing yarn,
c) wherein the braided construct contacting the outer surface of the balloon is characterized as the bonding yarn having been reflowed to mechanically connect to the polymeric material comprising the balloon and to the reinforcing yarn without the balloon and the reinforcing yarn having been melted.

11. A method for making a medical balloon, comprising the steps of:
a) providing a balloon comprising a base polymeric material of a first melting temperature;
b) braiding at least one bonding yarn of a second polymeric material having a second melting temperature with at least one reinforcing yarn of a third material having a third melting temperature to form a braided construct, wherein the second melting temperature of the second polymeric material comprising the bonding yarn is lower than the third melting temperature of the third material comprising the reinforcing yarn;
c) contacting the braided construct to the outer surface of the balloon to form a medical balloon assembly; and
d) heating the medical balloon assembly to a melting temperature that is greater than the second melting temperature of the second polymeric material comprising the bonding yarn but below the first and third melting temperatures of the base polymeric material of the balloon and the third material of the reinforcing yarn to cause the second polymeric material to melt and mechanically connect to the first polymeric material comprising the balloon and to the third material comprising the reinforcing yarn without the balloon and the reinforcing yarn having been melted.

12. The method of claim 11, including heating the medical balloon assembly to the melting temperature that is below the first melting temperature of the polymeric material comprising the balloon and the third melting temperature of the third material comprising the at least one reinforcing yarn.

13. The method of claim 11 or claim 12, including:
providing the braided construct as a braided sleeve and sliding the braided sleeve over the outer surface of the balloon prior to heating the medical balloon assembly; and/or
providing the at least one bonding yarn and the at least one reinforcing yarn in the braided construct in a ratio ranging from 1:2 to 1:4; and/or
orienting the at least one bonding yarn and the at least one reinforcing yarn with respect to each other in a range of from about 100° to about 140°.

14. The method of any of claims 11 to 13, including providing the balloon comprising an enlarged central balloon section meeting opposed tapered balloon sections extend axially to respective open-ended balloon sections, and further providing the braided construct in contact with the tapered balloon sections having an increased thickness in comparison to the braided construct in contact with the enlarged central balloon section and the open-ended balloon sections.

15. The method of any of claims 11 to 14, including:
selecting the base polymeric material comprising the balloon from a styrene-ethylene-butylene-styrene (SEBS) block copolymer, a styrene-isoprene-styrene (SIS) block copolymer, a styrene-butadiene-styrene (SBS) block copolymer, nylon, a poly (ether-block-amides, a poly(ester-block ether) elastomer, a poly(ester-block-ester) elastomer, a polyethylene terephthalate, and a polybutylene terephthalate; and/or
selecting the second polymeric material comprising the at least one bonding yarn from a copolyester designated GRILON KE-60 having a melting temperature of about 55°C. to about 65°C., a copolyester designated KE-60 having a melting temperature of about 60°C., a copolyimide designated K-85 having a melting temperature of about 85°C., a copolyester designated GRILON K-85 having a melting temperature of about 82°C. to about 92°C., a copolyimide designated K-110 having a melting temperature of about 110°C., a copolyester designated KE-135 having a melting temperature of about 135°C., a copolyimide designated K-140 having a melting temperature of about 140°C., a copolyester designated KE-160 having a melting temperature of about 160°C., Nylon 12 having a melting temperature of about 178°C., and PET (polyethylene terephthalate) having a melting temperature of about 260°C.
